# EUROPEAN PATENT SPECIFICATION

(11) **EP 4 594 278 B1**
(45) Date of publication and mention of the grant of the patent: **11.11.2026**
(21) Application number: 23828645.4
(22) Date of filing: 27.11.2023
(51) Int. Cl.: C07C 5/48, C07C 11/06, B01J 21/04

(54) **METHODS FOR PRODUCING OLEFINIC COMPOUNDS UTILIZING COMBUSTORS**
VERFAHREN ZUR HERSTELLUNG VON OLEFINISCHEN VERBINDUNGEN UNTER VERWENDUNG VON BRENNKAMMERN
PROCÉDÉS DE PRODUCTION DE COMPOSÉS OLÉFINIQUES À L'AIDE DE CHAMBRES DE COMBUSTION

(30) Priority: 29.11.2022 US 202263428525 P
(43) Date of publication of application: 06.08.2025
(73) Proprietor: Dow Global Technologies LLC, Midland, MI 48674 (US)
(72) Inventor: LUO, Lin, Freeport, Texas 77541 (US); WANG, Hangyao, Freeport, Texas 77541 (US); PRETZ, Matthew T., Freeport, Texas 77541 (US); YUAN, Quan, Lake Jackson, Texas 77566 (US); LI, Liwei, Freeport, Texas 77541 (US)
(74) Representative: Beck Greener LLP
(86) International application number: PCT/US2023/081077
(87) International publication number: WO 2024/118462

(56) References cited:
- WO-A1-2013/009820
- WO-A1-2020/009863
- US-A1- 2021 292 259

## Description

### CROSS-REFERENCE TO RELATED APPLICATIONS

This application claims the benefit of U.S. Provisional Application Serial No. 63/428,525 filed November 29, 2022.

### TECHNICAL FIELD

Embodiments described herein generally relate to chemical processing and, more specifically, to processes and systems utilized for the production of olefinic compounds.

### BACKGROUND

Olefinic compounds may be utilized as base materials to produce many types of goods and materials. For example, propylene may be utilized to manufacture polypropylene, propylene oxide, and acrylonitrile. Such products may be utilized in product packaging, chemical manufacturing, textiles, etc. Thus, there is an industry demand for olefinic compounds, such as ethylene, propylene, butene, and styrene, as well as processes to produce such materials. WO 2013/009820 discloses a process to increase propane dehydrogenation activity of a partially deactivated dehydrogenation catalyst by heating the partially deactivated catalyst to a temperature of at least 660°C, conditioning the heated catalyst in an oxygen-containing atmosphere and, optionally, stripping molecular oxygen from the conditioned catalyst.

### SUMMARY

One method for producing olefinic compounds is by dehydrogenating hydrocarbons. In some embodiments, the dehydrogenation reaction may use a particulate solid such as, for example, a catalyst to promote the dehydrogenation reaction. Further, because of the endothermic nature of the dehydrogenation reaction, the reaction may also utilize an external heat source to promote the reaction. In such embodiments, the particulate solid may be passed to a combustor where a supplemental fuel is reacted to heat the particulate solid. The heated particulate solid may provide some or all of the heat utilized to continue the dehydrogenation reaction. However, reacting the supplemental fuel in the presence of the particulate solid may reduce the activity of the particulate solid in promoting the dehydrogenation reaction. Accordingly, it would be desired to limit the amount of particulate solid exposed to the supplemental fuel while still providing a sufficient heat source for the dehydrogenation reaction. The methods of the present disclosure may help limit the amount of particulate solid exposed to the supplemental fuel by passing both deactivated and reactivated particulate solid into the combustor in a particular distribution pattern. Specifically, embodiments described herein utilize methods whereby deactivated particulate solid catalyst is passed into the combustor downstream of the supplemental fuel, and whereby reactivated particulate solid catalyst is passed into the combustor upstream of the supplemental fuel. Such an arrangement may be beneficial by increasing total catalytic activity in the reactor.

According to one or more embodiments of the present disclosure, a method of producing olefinic compounds may include contacting a feed stream including one or more hydrocarbons with a particulate solid catalyst in a reactor. In the reactor, the one or more hydrocarbons may be dehydrogenated to form one or more products including one or more olefinic compounds and at least a portion of the particulate solid catalyst may be deactivated. The method may also include passing at least a portion of the deactivated particulate solid catalyst to a combustor. In the combustor, a supplemental fuel stream may enter the combustor through a supplemental fuel distributor and the supplemental fuel stream may be reacted to heat at least a portion of the deactivated particulate solid catalyst. The method may also include passing at least a portion of the heated deactivated particulate solid catalyst to an oxygen treatment zone to produce a reactivated particulate solid catalyst. The method may also include passing at least a portion of the reactivated particulate solid catalyst back to the combustor. In the combustor, the deactivated particulate solid catalyst may enter the combustor downstream of the supplemental fuel stream relative to a flow direction of the supplemental fuel stream and the reactivated particulate solid catalyst may enter the combustor upstream of the supplemental fuel stream relative to the flow direction of the supplemental fuel stream. The method may also include passing at least a portion of the reactivated particulate solid catalyst to the reactor.

It is to be understood that both the preceding general description and the following detailed description describe various embodiments and are intended to provide an overview or framework for understanding the nature and character of the claimed subject matter. Additional features and advantages of the embodiments will be set forth in the detailed description and, in part, will be readily apparent to persons of ordinary skill in the art from that description, which includes the accompanying drawings and claims, or recognized by practicing the described embodiments. The drawings are included to provide a further understanding of the embodiments and, together with the detailed description, serve to explain the principles and operations of the claimed subject matter. However, the embodiments depicted in the drawings are illustrative and exemplary in nature, and not intended to limit the claimed subject matter.

### BRIEF DESCRIPTION OF THE DRAWINGS

The following detailed description may be better understood when read in conjunction with the following drawings, in which:
FIG. 1 schematically depicts a reactor system, according to one or more embodiments of the present disclosure;
FIG. 2 schematically depicts a combustor, according to one or more embodiments of the present disclosure; and
FIG. 3 graphically depicts example data, according to one or more embodiments of the present disclosure.

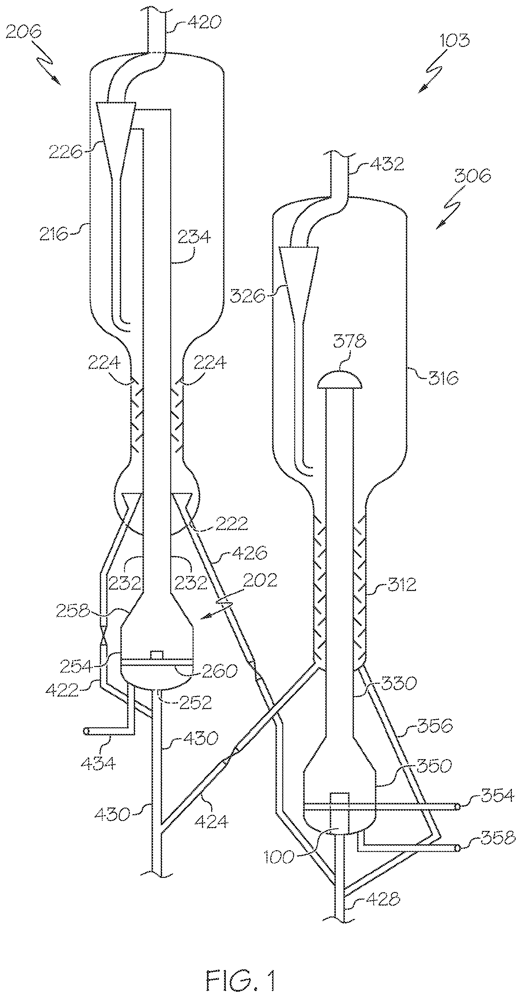

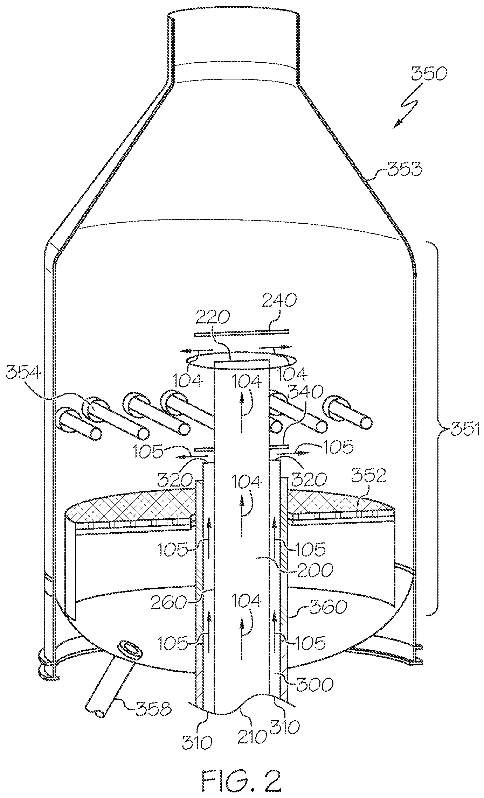

When describing the simplified schematic illustrations of FIG. 1 and FIG. 2 the numerous valves, temperature sensors, electronic controllers, and the like, which may be used and are well known to a person of ordinary skill in the art, are not included. Further, accompanying components that are often included in such reactor systems, such as air supplies, heat exchangers, surge tanks, and the like are also not included. However, it should be understood that these components are within the scope of the present disclosure

Reference will now be made in greater detail to various embodiments, some of which are illustrated in the accompanying drawings.

### DETAILED DESCRIPTION

Specific embodiments of the present application will now be described. The disclosure may, however, be embodied in different forms and should not be construed as limited to the embodiments set forth in this disclosure. Rather, these embodiments are provided so that this disclosure will be thorough and complete, and will fully convey the scope of the subject matter to those skilled in the art.

As described herein, methods for making for producing olefinic compounds may include a reactivating a catalyst in a combustor by exposure to a supplemental fuel. In embodiment described herein, deactivated particulate solid catalyst and recycled reactivated particulate solid catalyst may be separately passed to the combustor in a different regions of the combustor. As described herein, "particulate solid catalysts" refer to particulate solids that have catalytic functionality for dehydrogenation reactions and/or fuel combustion reactions. Where the term "particulate solid" is described herein, it may equally refer to a "particulate solid catalyst".

Embodiments of the methods presently disclosed will now be described herein in detail in the context of the reactor system of FIG. 1 and the combustor of FIG. 2 operating as a circulating fluidized bed to dehydrogenate hydrocarbons. However, it should be understood that the principles disclosed and taught herein may be applicable to other systems which utilize different system components oriented in different ways. For example, the concepts described herein may be equally applied to other systems with alternate reactor units and regeneration units, such as those that operate under non-fluidized conditions or include downers rather than risers, and vice versa. It should be further understood that not all portions of the reactor system of FIG. 1 and the combustor of FIG. 2 should be construed as essential to the claimed subject matter. Moreover, while the recited method steps in the appended claims are described herein in the context of the reactor systems of FIG. 1 and the combustor of FIG. 2, such recited method steps should be understood as adaptable to other systems, as would be understood by those skilled in the art.

Now referring to FIG. 1, an example reactor system 103 that may be suitable for use with the methods and/or apparatuses described herein is schematically depicted. The reactor system 103 generally comprises multiple system components, such as a reactor portion 206 and a regeneration unit 306. As described herein, "system components" refer to portions of the reactor system 103, such as reactors, separators, transfer lines, combinations thereof, and the like. As used herein in the context of FIG. 1, the reactor portion 206 generally refers to the portion of a reactor system 103 in which the major process reaction takes place (e.g., dehydrogenation) to form the product stream. A feed stream enters the reactor portion 206, is converted to a product stream (containing product and unreacted feed), and exits the reactor portion 206. The reactor portion 206 comprises a reactor 202, which may include an upstream reactor section 254 and a downstream reactor section 232. According to one or more embodiments, as depicted in FIG. 1, the reactor portion 206 may additionally include a particulate solid separation section 216, which serves to separate a particulate solid catalyst from the chemical products formed in the reactor 202. Also, as used herein, the regeneration unit 306 generally refers to the portion of the reactor system 103 where the particulate solid is in some way processed, such as by combustion, to, e.g., improve catalytic activity and/or heat the particulate solid. The regeneration unit 306 may comprise a combustor 350 and a riser 330, a particulate solid separation section 316, and may additionally comprise an oxygen treatment zone 312. In one or more embodiments, the particulate solid separation section 216 may be in fluid communication with the combustor 350 (e.g., via line 426) and the particulate solid separation section 316 may be in fluid communication with the upstream reactor section 254 (e.g., via line 424 and transport riser 430).

Generally, as is described herein, in embodiments illustrated in FIG. 1 a portion of the particulate solid is cycled between the reactor portion 206 and the regeneration unit 306. It should be understood that when particulate solids are referred to herein, they may refer to solid materials that are catalytically active for a desired reaction (i.e., catalysts), or may equally refer to other particulate solids referenced with respect to the system of FIG. 1 which do not necessarily have catalytic activity but affect the reaction, such as oxygen-carrier materials. The terms "catalytic activity" and "catalyst activity" refer to the degree to which a catalyst is able to catalyze the reactions conducted in the reactor system 103. The particulate solid that exits the reactor portion 206 may be deactivated particulate solid. As used herein, "deactivated" may refer to a particulate solid, which has reduced catalytic activity or is cooler as compared to particulate solid entering the reactor portion 206. However, deactivated particulate solid may maintain some catalytic activity. Reduced catalytic activity may result from contamination with a substance such as coke. Reactivation (sometimes called "regeneration" herein) may remove the contaminant such as coke, raise the temperature of the particulate solid, or both. In embodiments, deactivated particulate solid may be reactivated by particulate solid reactivation in the regeneration unit 306. The deactivated particulate solid may be reactivated by, but not limited to, removing coke by combustion, recovering catalyst acidity, oxidizing the particulate solid, heating the particulate solid, other reactivation process, or combinations thereof. In one or more embodiments, the particulate solid may be heated during reactivation by combustion of a supplemental fuel, such as hydrogen, methane, ethane, propane, natural gas, or combinations thereof. Without being bound by theory, it is believed that when the particulate solid is heated during reactivation by combustion of supplemental fuel the exposure of the particulate solid to fuel gas may deactivate the particulate solid, while still heating the particulate solid. As used herein the term "heated deactivated particulate solid catalyst" refers to a particulate solid catalyst that has been heated by combustion of a supplemental fuel, but that still may have reduced catalytic activity. The regenerated particulate solid from the regeneration unit 306 may then be passed back to the reactor portion 206.

The feed stream may enter feed inlet 434 into the reactor 202, and the product stream may exit the reactor system 103 via pipe 420. According to one or more embodiments, the reactor system 103 may be operated by feeding a chemical feed (e.g., in a feed stream) and a particulate solid into the upstream reactor section 254. The chemical feed contacts the particulate solid in the upstream reactor section 254, and each flow upwardly into and through the downstream reactor section 232 to produce a chemical product.

Now referring to FIG. 1 in detail, the reactor portion 206 may comprise an upstream reactor section 254, a transition section 258, and a downstream reactor section 232, such as a riser. The transition section 258 may connect the upstream reactor section 254 with the downstream reactor section 232. As depicted in FIG. 1, the upstream reactor section 254 may be positioned below the downstream reactor section 232. Such a configuration may be referred to as an upflow configuration in the reactor 202. The upstream reactor section 254 may include a vessel, drum, barrel, vat, or other container suitable for a given chemical reaction. As depicted in FIG. 1, the upstream reactor section 254 may be connected to the downstream reactor section 232 via the transition section 258. The upstream reactor section 254 may generally comprise a greater crosssectional area than the downstream reactor section 232. The transition section 258 may be tapered from the size of the cross-section of the upstream reactor section 254 to the size of the cross-section of the downstream reactor section 232 such that the transition section 258 projects inwardly from the upstream reactor section 254 to the downstream reactor section 232. For example, the transition section 258 may be a frustum.

The upstream reactor section 254 may be connected to a transport riser 430, which, in operation may provide regenerated particulate solid in a feed stream to the reactor portion 206. The particulate solid entering the upstream reactor section 254 via transport riser 430 may be passed through line 424 to a transport riser 430, thus arriving from the regeneration unit 306. The particulate solid may come directly from the particulate solid separation section 216 via standpipe 422 and into the transport riser 430, where it enters the upstream reactor section 254. This particulate solid may be somewhat deactivated, but may still, in some embodiments, be suitable for reaction in the upstream reactor section 254, particularly when used in combination with the regenerated/reactivated particulate solid.

Still referring to FIG. 1, in one or more embodiments, based on the shape, size, and other processing conditions (such as temperature and pressure) in the upstream reactor section 254 and the downstream reactor section 232, the upstream reactor section 254 may operate as a fluidized bed, such as in a fast fluidized, turbulent, or bubbling bed upflow reactor, while the downstream reactor section 232 may operate in more of a plug flow manner, such as in a riser reactor. For example, the reactor 202 of FIG. 1 may comprise an upstream reactor section 254 operating as a fast fluidized, turbulent, or bubbling bed reactor and a downstream reactor section 232 operating as a dilute phase riser reactor, with the result that the average particulate solid and gas flow moves concurrently upward. As the term is used herein, "average flow" refers to the net flow, i.e., the total upward flow minus the retrograde or reverse flow, as is typical of the behavior of fluidized particles in general. As described herein, a "fast fluidized" reactor may refer to a reactor utilizing a fluidization regime wherein the superficial velocity of the gas phase is greater than the choking velocity and may be semi-dense in operation. As described herein, a "turbulent" reactor may refer to a fluidization regime where the superficial velocity of less than the choking velocity and is more dense than the fast fluidized regime. As described herein, a "bubbling bed" reactor may refer to a fluidization regime wherein well defined bubbles in a highly dense bed are present in two distinct phases. The "choking velocity" refers to the minimum velocity required to maintain solids in the dilute-phase mode in a vertical conveying line. As described herein, a "dilute phase riser" may refer to a riser reactor operating at transport velocity, where the gas and particulate solid have about the same velocity in a dilute phase.

In one or more embodiments, the particulate solid may be capable of fluidization. In some embodiments, the particulate solid may exhibit properties known in the industry as "Geldart A" or "Geldart B" properties. Particles may be classified as "Group A" or "Group B" according to D. Geldart, Gas Fluidization Technology, John Wiley & Sons (New York, 1986), 34-37; and D. Geldart, "Types of Gas Fluidization," Powder Technol. 7 (1973) 285-292.

Group A is understood by those skilled in the art as representing an aeratable powder, having a bubble-free range of fluidization; a high bed expansion; a slow and linear deaeration rate; bubble properties that may include a predominance of splitting/recoalescing bubbles, with a maximum bubble size and large wake; high levels of solids mixing and gas backmixing, assuming equal U-Umf (U is the velocity of the carrier gas, and Umf is the minimum fluidization velocity, typically though not necessarily measured in meters per second, m/s, i.e., there is excess gas velocity); axisymmetric slug properties; and no spouting, except in very shallow beds. The properties listed tend to improve as the mean particle size decreases, assuming equal cfp; or as the <45 micrometers (µm) proportion is increased; or as pressure, temperature, viscosity, and density of the gas increase. In general, the particles may exhibit a small mean particle size and/or low particle density (<1.4 grams per cubic centimeter, g/cm³), fluidize easily, with smooth fluidization at low gas velocities, and may exhibit controlled bubbling with small bubbles at higher gas velocities.

Group B is understood by those skilled in the art as representing a "sand-like" powder that starts bubbling at Umf; that exhibits moderate bed expansion; a fast deaeration; no limits on bubble size; moderate levels of solids mixing and gas backmixing, assuming equal U-Umf; both axisymmetric and asymmetric slugs; and spouting in only shallow beds. These properties tend to improve as mean particle size decreases, but particle size distribution and, with some uncertainty, pressure, temperature, viscosity, or density of gas seem to do little to improve them. In general, most of the particles having a particle size (cfp) of 40 µm <cfp <500 µm when the density (pp) is 1.4 <pp <4 g/cm³, and preferably 60 µm <cfp <500 µm when the density (pp) is 4 g/cm³ and 250 µm <cfp <100 µm when the density (pp) is 1 g/cm³.

According to embodiments, the chemical product and the particulate solid may be passed out of the downstream reactor section 232 to a separation device 226 in the particulate solid separation section 216, where the particulate solid is separated from the chemical product, which is transported out of the particulate solid separation section 216. According to one or more embodiments, following separation from vapors in the separation device 226, the particulate solid may generally move through the strip zone 224 to the particulate solid outlet port 222 where the particulate solid is transferred out of the reactor portion 206 via line 426 and into the regeneration unit 306.

Now referring back to FIG. 1, according to one or more embodiments, the separation device 226 may be a cyclonic separation system, which may include two or more stages of cyclonic separation. In embodiments where the separation device 226 comprises more than one cyclonic separation stages, the first separation device into which the fluidized stream enters is referred to as a primary cyclonic separation device. The fluidized effluent from the primary cyclonic separation device may enter into a secondary cyclonic separation device for further separation. Primary cyclonic separation devices may include, for example, primary cyclones, and systems commercially available under the names VSS (commercially available from UOP), LD2 (commercially available from Stone and Webster), and RS2 (commercially available from Stone and Webster). Primary cyclones are described, for example, in U.S. Patent Nos. 4,579,716; 5,190,650; and 5,275,641. In some separation systems utilizing primary cyclones as the primary cyclonic separation device, one or more set of additional cyclones, e.g. secondary cyclones and tertiary cyclones, are employed for further separation of the particulate solid from the product gas. It should be understood that any primary cyclonic separation device may be used in embodiments of the invention.

Referring still to FIG. 1, the separated particulate solid is passed from the particulate solid separation section 216 to the combustor 350 via line 426. In the combustor 350, the particulate solid may be processed by, for example, combustion with one or both of oxygen and a supplemental fuel. For example, and without limitation, the particulate solid may be de-coked and/or fuel may be combusted to heat the particulate solid. The particulate solid is then passed out of the combustor 350 and through the riser 330 to a riser termination separator 378, where the gas and solid components from the riser 330 are at least partially separated. The vapor and remaining solids are transported to a secondary separation device 326 in the particulate solid separation section 316 where the remaining particulate solid is separated from the gases from the particulate solid processing (e.g., gases emitted by combustion of coke on spent particulate solid or fuel, referred to herein as flue gas). The flue gas may pass out of the regeneration unit 306 via outlet pipe 432. The separated particulate solid is then passed through the oxygen treatment zone 312 within the particulate solid separation section 316 to the upstream reactor section 254 via line 424 and transport riser 430, where it is further utilized in a catalytic reaction. In one or more embodiments, at least a portion of the particulate solid may be passed from the oxygen treatment zone 312 to the combustor 350. Thus, the particulate solid, in operation, may cycle between the reactor portion 206 and the regeneration unit 306. In general, the processed chemical streams, including the feed streams and product streams may be gaseous, and the particulate solid may be fluidized.

Referring now to the regeneration unit 306, as depicted in FIG. 1, the combustor 350 of the regeneration unit 306 may be in fluid communication with the riser 330. Oxygen-containing gas, such as air, may be passed through pipe 358 into the combustor 350. The combustor 350 may be in fluid communication with the particulate solid separation section 216 via line 426, which may supply deactivated particulate solid from the reactor portion 206 to the regeneration unit 306 for regeneration. The combustor 350 may also be in fluid communication with oxygen treatment zone 312, which may supply reactivated particulate solid to the reactor portion 206 and the combustor 350. The combustor 350 and riser 330 may operate with similar or identical fluidization regimes as to what was disclosed with respect to the upstream reactor section 254 and downstream reactor section 232 of the reactor portion 206. That is, the combustor 350 may operate as a fluidized bed, such as in a fast fluidized, turbulent, or bubbling bed upflow reactor, while the riser 330 may operate in more of a plug flow manner, such as in a riser reactor. Geometries as described with respect to the upstream reactor section 254 and downstream reactor section 232 may equally apply to the combustor 350 and riser 330. Additionally, the combustor 350 may also include a supplemental fuel distributor 354, which may supply a fuel, such as a hydrocarbon stream, hydrogen, or a mixture to the combustor 350.

The particulate solid and flue gas produced in the combustor 350 may travel through riser 330 to the particulate solid separation section 316. In the particulate solid separation section 316 the flue gas and the particulate solid may be separated first by a riser termination separator 378 and then by a secondary separation device 326. As described in one or more embodiments, following separation of flue gas from the particulate solid in the riser termination separator 378 and secondary separation device 326, treatment of the processed particulate solid with an oxygen-containing gas is conducted in the oxygen treatment zone 312. In some embodiments, the oxygen treatment zone 312 includes a fluid solids contacting device. The fluid solids contacting device may include baffles or grid structures to facilitate contact of the processed catalyst with the oxygen-containing gas. Examples of fluid solid contacting devices are described in further detail in U.S. Patent Nos. 9,827,543 and 9,815,040. The fluidization regime within the oxygen treatment zone may be bubbling bed type fluidization.

In one or more embodiments, the particulate solid may be exposed to the oxygen-containing gas in the oxygen treatment zone 312 for from 30 seconds to 20 minutes. For example, the particulate solid may be exposed to the oxygen-containing gas in the oxygen treatment zone 312 for from 30 seconds to 18 minutes, such as from 30 seconds to 16 minutes, from 30 seconds to 14 minutes, from 30 seconds to 12 minutes, from 30 seconds to 10 minutes, from 30 seconds to 8 minutes, from 30 seconds to 6 minutes, from 30 seconds to 4 minutes, from 30 seconds to 2 minutes, from 2 minutes to 20 minutes, from 2 minutes to 18 minutes, from 2 minutes to 16 minutes, from 2 minutes to 14 minutes, from 2 minute to 12 minutes, from 2 minutes to 10 minutes, from 2 minutes to 8 minutes, from 2 minutes to 6 minutes, from 2 minutes to 4 minutes, from 4 minutes to 20 minutes, from 4 minutes to 18 minutes, from 4 minutes to 16 minutes, from 4 minutes to 14 minutes, from 4 minutes to 12 minutes, from 4 minutes to 10 minutes, from 4 minutes to 8 minutes, from 4 minutes to 6 minutes, from 6 minutes to 20 minutes, from 6 minutes to 18 minutes, from 6 minutes to 16 minutes, from 6 minutes to 14 minutes, from 6 minutes to 12 minutes, from 6 minutes to 10 minutes, from 6 minutes to 8 minutes, from 8 minutes to 20 minutes, from 8 minutes to 18 minutes, from 8 minutes to 16 minutes, from 8 minutes to 14 minutes, from 8 minutes to 12 minutes, from 8 minutes to 10 minutes, from 10 minutes to 20 minutes, from 10 minutes to 18 minutes, from 10 minutes to 16 minutes, from 10 minutes to 14 minutes, from 10 minutes to 12 minutes, from 12 minutes to 20 minutes, from 12 minutes to 18 minutes, from 12 minutes to 16 minutes, from 12 minutes to 14 minutes, from 14 minutes to 20 minutes, from 14 minutes to 18 minutes, from 14 minutes to 16 minutes, from 16 minutes to 20 minutes, from 16 minutes to 18 minutes, or from 18 minutes to 20 minutes.

In one or more embodiments, a portion of the particulate solid may be passed through the oxygen treatment zone 312 and passed back to the reactor portion 206 via line 424. In one or more embodiments, a portion of the particulate solid may be passed through the oxygen treatment zone 312 and passed back to the combustor 350 via line 356 and pipe 428. Pipe 428 may separately carry the portion of particulate solid coming from the oxygen treatment zone 312 via line 356 and the portion of particulate solid coming from the particulate solid separation section 216 via line 426 to the particulate solids distributor 100, which may be operable to pass the two particulate solid portions separately into the combustor 350.

In one or more embodiments, at least a portion of the particulate solid may be removed from the oxygen treatment zone 312 after having passed through only part of the oxygen treatment zone. For example, if the oxygen treatment zone 312 exposed the particulate solid to the oxygen-containing gas for 5 minutes, then a portion of the particulate solid may be removed from the oxygen-treatment zone after having been exposed to the oxygen-containing gas for only 1 minute. In one or more embodiments, the portion of the particulate solid removed from the oxygen treatment zone 312 without having passed through the entire oxygen treatment zone 312 may be passed to the combustor 350 as reactivated particulate solid (not shown in FIG. 1).

Referring now to FIG. 2, an example combustor 350 that may be suitable for use with the methods described herein is schematically depicted. FIG. 2 shows a combustor 350 used as a fluidized fuel gas combustor system for a dehydrogenation process. However, the methods described herein may be used with a variety of combustor systems as would be understood by those skilled in the art. The combustor 350 may include a lower portion 351 generally in the shape of a cylinder and an upper portion comprising a frustum 353. The angle between the frustum 353 and an internal horizontal imaginary line drawn at the intersection of the frustum 353 and the lower portion 351 may range from 10 to 80 degrees. All individual values and subranges from 10 to 80 degrees are included and disclosed herein; for example, the angle between the tubular and frustum 353 components can range from a lower limit of 10, 40 or 60 degrees to an upper limit of 30, 50, 70 or 80 degrees. For example, the angle can be from 10 to 80 degrees, or in the alternative, from 30 to 60 degrees, or in the alternative, from 10 to 50 degrees, or in the alternative, from 40 to 80 degrees. Furthermore, in alternative embodiments, the angle can change along the height of the frustum 353, either continuously or discontinuously. In some embodiments, the combustor 350 may be, or may not be, lined with a refractory material.

In one or more embodiments, a supplemental fuel stream may enter the combustor 350 through a supplemental fuel distributor 354. In one or more embodiments, the supplemental fuel stream may comprise hydrogen, methane, ethane, propane, natural gas, or combinations thereof. In the combustor 350, the supplemental fuel may react with oxygen and/or with the particulate solid. Without being bound by theory, it is believed that the combustion of the supplemental fuel in the combustor 350 may heat the particulate solid. Aside from the combustion of supplemental fuel, coke from the particulate solid may also combust in the combustor 350, which may heat the particulate solids and re-activate the particulate solid. In some embodiments, oxygen treatment in oxygen treatment zone 312 further re-activates the particulate solid.

Deactivated particulate solid 104 (e.g., from line 426 in FIG. 1) and reactivated particulate solid 105 (e.g., from line 356 in FIG. 1, and as described hereinafter) may enter the combustor 350 through a particulate solids distributor 100. Deactivated particulate solid 104 and reactivated particulate solid 105 may be passed separately through the particulate solids distributor 100 and into the combustor 350. In embodiments, the deactivated particulate solid 104 and the reactivated particulate solid 105 are not mixed prior to entering the combustor 350. In one or more embodiments, the reactivated particulate solid 105 may enter the combustor 350 upstream of the supplemental fuel stream relative to a flow direction of the supplemental fuel stream and the deactivated particulate solid 104 may enter the combustor downstream of the supplemental fuel stream relative to the flow direction of the supplemental fuel stream. In some such embodiments, the supplemental fuel stream may first combust and contact the reactivated particulate solid 105 before any residual supplemental fuel can contact the deactivated particulate solid 104. In such embodiments, the supplemental fuel stream may first contact the reactivated particulate solid 105 before any remaining (non-combusted) supplemental fuel may contact the deactivated particulate solid 104. Generally, if any residual supplemental fuel contacts the deactivated particulate solid 104, it is in much lower concentrations that when it contacts the reactivated particulate solid 105.

As shown in FIG. 2, in one or more embodiments, the particulate solids distributor 100 may extend into the combustor 350 through a bottom end of the combustor 350. In one or more embodiments, the particulate solids distributor 100 may pass the deactivated particulate solid 104 into the combustor 350 above the supplemental fuel distributor 354 and may pass the reactivated particulate solid 105 into the combustor 350 below the supplemental fuel distributor 354.

FIG. 2 depicts one contemplated particulate solids distributor. However, other solids distributors may be suitable, and the embodiments described herein should not be construed as limited by the design, shape, size, architecture, etc. of the distributor or distributors that pass particulate solids into the combustor 350.

As shown in FIG. 2, in one or more embodiments, the particulate solids distributor 100 may include an inner conduit 200 and an outer conduit 300. The inner conduit 200 may extend from an inner conduit inlet 210 to an inner conduit outlet 220. The inner conduit 200 may be at least partially defined by an inner wall 260. The inner wall 260 may be arranged around a central axis. The outer conduit 300 may extend from an outer conduit inlet 310 to an outer conduit outlet 320. The outer conduit 300 may be at least partially defined by the inner wall 260 and an outer wall 360. The outer wall 360 may be arranged around the central axis and a cross-section of the outer wall 360 may surround a cross-section of the inner wall 260 in a plane perpendicular to the central axis. The particulate solids distributor 100 may also include a first solids director 240 and a second solids director 340. In some embodiments, as shown in FIG. 2, the first solids director 240 and the second solids director 340 may be a first deflector plate and a second deflector plate.

The deactivated particulate solid 104 may be passed into the inner conduit 200 through inner conduit inlet 210 and may pass through the inner conduit 200 and out inner conduit outlet 220 to the first solids director 240, which may direct the deactivated particulate solid 104 into the combustor 350. The reactivated particulate solid 105 may be passed into the outer conduit 300 through outer conduit inlet 310 and may pass through the outer conduit 300 and out outer conduit outlet 320 to the second solids director 340, which may direct the reactivated particulate solid 105 into the combustor 350.

Still referring to FIG. 2, as the supplemental fuel enters the combustor 350 via supplemental fuel distributor 354 it contacts an oxygen-containing gas, which enters the combustor upstream of the supplemental fuel stream relative to the flow direction of the supplemental fuel stream via pipe 358, causing combustion of the supplemental fuel. The combustor 350 may include a grid distributor 352 that evenly distributes the oxygen-containing gas across the surface of the gird distributor 352. In one or more embodiments, the grid distributor 352 is not connected to the outer wall 360 of the particulate solids distributor 100. As the supplemental fuel distributor 354 is downstream of pipe 358, the supplemental fuel entering the combustor 350 contacts the oxygen-containing gas upon entering the combustor 350. Accordingly, the concentration of supplemental fuel in the combustor 350 decreases as the supplemental fuel travels up through the combustor 350 and away from the supplemental fuel distributor 354 toward the frustum 353. In one or more embodiments, the concentration of supplemental fuel in the combustor 350 may be lower in the area where the deactivated particulate solid 104 enters the combustor 350 when compared to the concentration of supplemental fuel in the area where the reactivated particulate solid 105 enters the combustor 350. In some embodiments, greater than or equal to 80% of the supplemental fuel may be combusted in the area below where the deactivated particulate solid 104 enters the combustor. For example, greater than or equal to 85% of the supplemental fuel may be combusted before the deactivated particulate solid 104 enters the combustor, such as greater than or equal to 90%, greater than or equal to 95%, or even greater than or equal to 99% of the supplemental fuel in the supplemental fuel stream.

Without being bound by theory, it is believed that by adding deactivated particulate solid 104 to the combustor 350 downstream of the supplemental fuel stream relative to a flow direction of the supplemental fuel stream and adding reactivated particulate solid 105 to the combustor 350 upstream of the supplemental fuel stream relative to the flow direction of the supplemental fuel stream, total catalytic activity in the reactor may be increased. In particular, in one or embodiments, exposure to combustion of supplemental fuel may cause deactivation in the particulate solid catalyst. As shown in the Examples that follow, in some embodiments deactivation by combustion of supplemental fuels such as methane is non-linear. That is, methane concentration deactivates catalysts, but does so where very high amounts of methane cause increased deactivation in a non-linear relationship. That being such, in some embodiments, exposing the activated catalyst to the supplemental fuel will have less overall deactivation impact than exposing the deactivated catalyst to the supplemental fuel. For example, identical exposures to supplemental fuel combustion by methane may cause X amount of deactivation for dehydrogenation on a reactivated catalyst, and Y amount of deactivation for dehydrogenation on a deactivated catalyst, where X is less than Y. In such embodiments, majorly exposing the reactivated catalyst to the supplemental fuel combustion can improve overall efficiencies since the combination of reactivated and deactivated catalyst may have greater overall catalytic activity for dehydrogenation.

Without being bound by theory, it is further believed that because the heat transfer from one particle of particulate solid to another particle of particulate solid is rapid, the heat gained by combusting supplemental fuel may be transferred throughout the mass of particulate solid in the combustor without requiring every particle of particulate solid to be exposed to the supplemental fuel and supplemental fuel combustion. This may allow only a portion of the particulate solid to be exposed to the supplemental fuel and supplemental fuel combustion while still heating the total mass of the particulate solid for use in the dehydrogenation reaction. Passing deactivated particulate solid 104 into the combustor 350 downstream of the supplemental fuel stream and passing reactivated particulate solid 105 into the combustor 350 may allow less of the particulate solid to contact the supplemental fuel stream while still sufficiently heating the entire mass of particulate solid within the combustor 350 which may, for example, improve the lifespan of the particulate solid and the dehydrogenation activity of the particulate solid.

In one or more embodiments, the deactivated particulate solid 104 passed to the combustor 350 from the reactor portion 206 may have a temperature of from 580 °C to 800 °C. For example, the deactivated particulate solid 104 passed to the combustor 350 from the reactor portion 206 may have a temperature that is from 580 °C to 775 °C, such as from 580 °C to 750 °C, from 580 °C to 725 °C, from 580 °C to 700 °C, from 580 °C to 675 °C, from 580 °C to 650 °C, from 580 °C to 625 °C, from 580 °C to 600 °C, from 600 °C to 800 °C, from 600 °C to 775 °C, from 600 °C to 750 °C, from 600 °C to 725 °C, from 600 °C to 700 °C, from 600 °C to 675 °C, from 600 °C to 650 °C, from 600 °C to 625 °C, from 625 °C to 800 °C, from 625 °C to 775 °C, from 625 °C to 750 °C, from 625 °C to 725 °C, from 625 °C to 700 °C, from 625 °C to 675 °C, from 625 °C to 650 °C, from 650 °C to 800 °C, from 650 °C to 775 °C, from 650 °C to 750 °C, from 650 °C to 725 °C, from 650 °C to 700 °C, from 650 °C to 675 °C, from 675 °C to 800 °C, from 675 °C to 775 °C, from 675 °C to 750 °C, from 675 °C to 725 °C, from 675 °C to 700 °C, from 700 °C to 800 °C, from 700 °C to 775 °C, from 700 °C to 750 °C, from 700 °C to 725 °C, from 725 °C to 800 °C, from 725 °C to 775 °C, from 725 °C to 750 °C, from 750 °C to 800 °C, from 750 °C to 775 °C, from 775 °C to 800 °C, or any combination of these ranges.

In one or more embodiments, the reactivated particulate solid 105 passed to the combustor 350 from the oxygen treatment zone 370 may have a temperature of from 680 °C to 900 °C. For example, the reactivated particulate solid passed 105 to the combustor 350 from the reactor portion may have a temperature that is from 680 °C to 875 °C, such as from 680 °C to 850 °C, from 680 °C to 825 °C, from 680 °C to 800 °C, from 680 °C to 775 °C, from 680 °C to 750 °C, from 680 °C to 725 °C, from 680 °C to 700 °C, from 700 °C to 900 °C, from 700 °C to 875 °C, from 700 °C to 850 °C, from 700 °C to 825 °C, from 700 °C to 800 °C, from 700 °C to 775 °C, from 700 °C to 750 °C, from 700 °C to 725 °C, from 725 °C to 900 °C, from 725 °C to 875 °C, from 725 °C to 850 °C, from 725 °C to 825 °C, from 725 °C to 800 °C, from 725 °C to 775 °C, from 725 °C to 750 °C, from 750 °C to 900 °C, from 750 °C to 875 °C, from 750 °C to 850 °C, from 750 °C to 825 °C, from 750 °C to 800 °C, from 750 °C to 775 °C, from 775 °C to 900 °C, from 775 °C to 875 °C, from 775 °C to 850 °C, from 775 °C to 825 °C, from 775 °C to 800 °C, from 800 °C to 900 °C, from 800 °C to 875 °C, from 800 °C to 850 °C, from 800 °C to 825 °C, from 825 °C to 900 °C, from 825 °C to 875 °C, from 825 °C to 850 °C, from 850 °C to 900 °C, from 850 °C to 875 °C, from 875 °C to 900 °C, or any combination of these ranges.

In non-limiting examples, the reactor system 103 described herein may be utilized to produce olefinic compounds from hydrocarbon feed streams. As used herein, the term "olefinic compounds" refers to hydrocarbons having one or more carbon-carbon double bonds apart from the formal double bonds in aromatic compounds. For example, ethylene and styrene are olefinic compounds, but ethylbenzene would not be an olefinic compound as the only double bonds present in ethylbenzene are formal double bonds present as part of the aromatic structure. Olefinic compounds may be produced from a variety of hydrocarbon feed streams by utilizing different reaction mechanisms. For example, olefinic compounds may be produced by at least dehydrogenation reactions, cracking reactions, dehydration reactions, and methanol-to-olefin reactions. These reaction types may utilize different feed streams and different particulate solid catalysts to produce olefinic compounds. It should be understood that when "catalysts" are referred to herein, they may equally refer to the particulate solid catalysts referenced with respect to the system of FIG. 1.

According to one or more embodiments, the reaction may be a dehydrogenation reaction. According to such embodiments, the one or more hydrocarbons may be a hydrocarbon feed stream the hydrocarbon feed stream may comprise one or more of ethylbenzene, ethane, propane, n-butane, and i-butane. In one or more embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of ethylbenzene. In one or more embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of ethane. In additional embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of propane. In additional embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of n-butane. In additional embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of i-butane. In additional embodiments, the hydrocarbon feed stream may comprise at least 50 wt.%, at least 60 wt.%, at least 70 wt.%, at least 80 wt.%, at least 90 wt.%, at least 95 wt.% or even at least 99 wt.% of the sum of ethylbenzene, ethane, propane, n-butane, and i-butane.

In one or more embodiments, the dehydrogenation reaction may utilize gallium and/or platinum particulate solids as a catalyst. In such embodiments, the particulate solids may comprise a gallium and/or platinum catalyst. As described herein, a gallium and/or platinum catalyst comprises gallium, platinum, or both. The gallium and/or platinum catalyst may be carried by an alumina or alumina silica support, and may optionally comprise potassium. Such gallium and/or platinum catalysts are disclosed in U.S. Pat. No. 8,669,406. However, it should be understood that other suitable catalysts may be utilized to perform the dehydrogenation reaction.

In one or more embodiments, the reaction mechanism may be dehydrogenation followed by combustion (in the same chamber). In such embodiments, a dehydrogenation reaction may produce hydrogen as a byproduct, and an oxygen carrier material may contact the hydrogen and promote combustion of the hydrogen, forming water. Examples of such reaction mechanisms, which are contemplated as possible reactions mechanisms for the systems and methods described herein, are disclosed in WO 2020/046978 and U.S. Pat. Pub. No. 2021/0292259.

In one or more embodiments, the particulate solid catalyst may comprise an oxygen-carrier material and a dehydrogenation catalyst material. In some embodiments, the oxygen-carrier material and the dehydrogenation catalyst material may be separate particles of the particulate solid. In some embodiments, the oxygen-carrier material and the dehydrogenation catalyst may be contained in the same particles of the particulate solid.

In some embodiments, the particulate solid catalyst may comprise a "dual-purpose material" that may act as both a dehydrogenation catalyst as well as an oxygen-carrier material. It should be understood that, in at least the embodiments described herein where an oxygen-carrier material and a dehydrogenation catalyst are utilized in the same reaction vessel (such as those of FIG. 1), such a dual-purpose material may be utilized either in replacement or in combination with the oxygen-carrier material of the particulate solid or the dehydrogenation catalyst of the particulate solid.

In one or more embodiments, the olefinic compounds may be present in a "product stream" sometimes called an "olefin-containing effluent". Such a stream exits the reactor system of FIG. 1 and may be subsequently processed. In one or more embodiments, the olefinic compounds may comprise one or more of ethylene, propylene, butylene, or styrene. The term butylene includes any isomers of butylene, such as α-butylene, cis-β-butylene, trans-β-butylene, and isobutylene. In some embodiments, the olefin-containing effluent may comprise at least 20 wt. %, at least 30 wt. %, at least 40 wt. %, at least 50 wt. %, or even at least 60 wt. % of ethylene. In additional embodiments, the olefin-containing effluent may comprise at least 20 wt. %, at least 30 wt. %, at least 40 wt. %, at least 50 wt. %, or even at least 60 wt. % of propylene. In additional embodiments, the olefin-containing effluent may comprise at least 20 wt. %, at least 30 wt. %, at least 40 wt. %, at least 50 wt. %, or even at least 60 wt. % of butylene. In additional embodiments, the olefin-containing effluent may comprise at least 20 wt. %, at least 30 wt. %, at least 40 wt. %, at least 50 wt. %, or even at least 60 wt. % of styrene. In additional embodiments, the olefin-containing effluent may comprise at least 20 wt. %, at least 30 wt. %, at least 40 wt. %, at least 50 wt. %, or even at least 60 wt. % of the sum of one or more of ethylene, propylene, butylene, and styrene. The olefin-containing effluent may further comprise unreacted components of the feed stream, as well as other reaction products that are not considered light olefins. The olefinic compounds may be separated from unreacted components in subsequent separation steps.

In a first aspect of the present disclosure, a method of producing olefinic compounds may include contacting a feed stream including one or more hydrocarbons with a particulate solid catalyst in a reactor. In the reactor, the one or more hydrocarbons may be dehydrogenated to form one or more products including one or more olefinic compounds and at least a portion of the particulate solid catalyst may be deactivated. The method may also include passing at least a portion of the deactivated particulate solid catalyst to a combustor. In the combustor, a supplemental fuel stream may enter the combustor through a supplemental fuel distributor and the supplemental fuel stream may be reacted to heat at least a portion of the deactivated particulate solid catalyst. The method may also include passing at least a portion of the heated deactivated particulate solid catalyst to an oxygen treatment zone to produce a reactivated particulate solid catalyst. The method may also include passing at least a portion of the reactivated particulate solid catalyst back to the combustor. In the combustor, the deactivated particulate solid catalyst may enter the combustor downstream of the supplemental fuel stream relative to a flow direction of the supplemental fuel steam and the reactivated particulate solid catalyst may enter the combustor upstream of the supplemental fuel stream relative to the flow direction of the supplemental fuel stream. The method may also include passing at least a portion of the reactivated particulate solid catalyst to the reactor.

A second aspect of the present disclosure includes any previous aspect or combination of aspects, where the reactivated particulate solid catalyst and the deactivated particulate solid catalyst are passed to the combustor through a particulate solids distributor that separately passes the reactivated particulate solid catalyst and deactivated particulate solid catalyst into the combustor.

A third aspect of the present disclosure includes any previous aspect or combination of aspects, where the particulate solids distributor extends into the reactor through a bottom end of the reactor.

A fourth aspect of the present disclosure includes any previous aspect or combination of aspects, where the particulate solids distributor passes the deactivated particulate solid catalyst into the combustor above the supplemental fuel distributor and passes the reactivated particulate solid catalyst into the combustor below the supplemental fuel distributor.

A fifth aspect of the present disclosure includes any previous aspect or combination of aspects, where the combustor operates as a fast fluidized, turbulent, or bubbling bed.

A sixth aspect of the present disclosure includes any previous aspect or combination of aspects, where the temperature of the deactivated particulate solid catalyst passed to the combustor is from 580 °C to 800 °C.

A seventh aspect of the present disclosure includes any previous aspect or combination of aspects, where the temperature of the reactivated particulate solid catalyst passed to the combustor is from 680 °C to 900 °C.

An eighth aspect of the present disclosure includes any previous aspect or combination of aspects, where in the oxygen treatment zone the heated deactivated catalyst is exposed to an oxygen-containing gas

A ninth aspect of the present disclosure includes any previous aspect or combination of aspects, where the heated deactivated particulate solid catalyst is exposed to the oxygen-containing gas for from 30 seconds to 20 minutes.

A tenth aspect of the present disclosure includes any previous aspect or combination of aspects, where the particulate solid catalyst comprises one or both of a dehydrogenation catalyst material and an oxygen-carrier material.

An eleventh aspect of the present disclosure includes any previous aspect or combination of aspects, where the dehydrogenation catalyst material and the oxygen-carrier material are contained in the same particles of the particulate solid catalyst.

A twelfth aspect of the present disclosure includes any previous aspect or combination of aspects, where the supplemental fuel stream comprises hydrogen, methane, ethane, propane, natural gas or combinations thereof.

A thirteenth aspect of the present disclosure includes any previous aspect or combination of aspects, where coke forms on the deactivated particulate solid catalyst in the reactor and at least a portion of the coke on the deactivated particulate solid catalyst is reacted in the combustor.

A fourteenth aspect of the present disclosure includes any previous aspect or combination of aspects, where the particulate solid catalyst is a Geldart A or Geldart B particulate.

A fifteenth aspect of the present disclosure includes any previous aspect or combination of aspects, where the one or more hydrocarbons comprise propane and the one or more olefinic compounds comprise propylene.

### EXAMPLES

Embodiments of the present disclosure will be further clarified by the following non-limiting examples.

### Example 1: Effect of Methane Exposure on Catalytic Activity

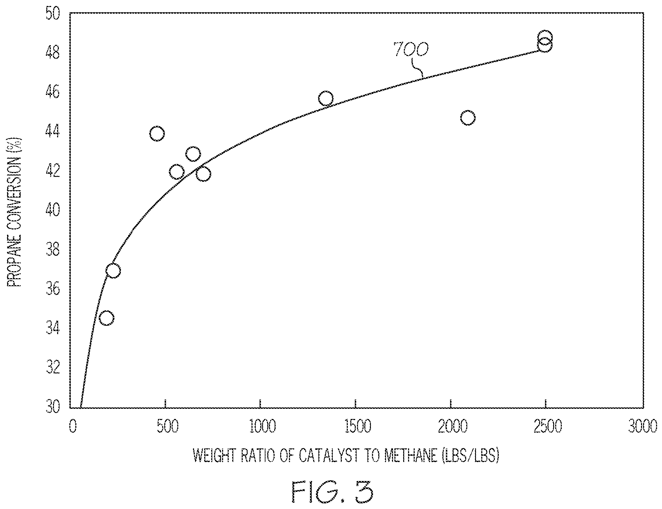

Referring to FIG. 3, propane conversion (%) (y-axis) (700) is shown graphically as a function of the weight ratio of catalyst to methane in the combustor (x-axis) for. As shown graphically in FIG. 3, the propane conversion 700 increases as the weight ratio of catalyst to methane in the combustor increases given that the total heat input to the reactor system is held constant.

As shown in FIG. 3, the propane conversion increases rapidly when the weight ratio of catalyst to methane in the combustor is increased up to about 300:1. In other words, below a catalyst to methane weight ratio of 300:1, the propane conversion decreases rapidly. Therefore, operating the reactor system with a catalyst to methane weight ratio in the combustor of greater than or equal to 300:1 provides superior performance compared to operating the reactor system with a catalyst to methane weight ratio of less than 300:1. As the weight ratio of catalyst to methane in the combustor is increased above 300:1, such as from 300:1 to 2500:1, the rate of increase in the propane conversion becomes slower and eventually reaches a plateau level at slightly less than 50% conversion, at which point the amount of methane (hydrocarbon) in the combustor is negligible. In FIG. 3, a catalyst to methane weight ratio of about 2500:1 is assumed to represent an infinitely large weight ratio corresponding to no methane in the combustor (e.g., pure hydrogen fuel without methane or other hydrocarbons in the supplemental fuel stream). FIG. 3 demonstrates that methane concentration deactivates catalysts, but does so where very high amounts of methane cause increased deactivation in a non-linear relationship. Accordingly, operating the combustor to limit the total exposure of the catalyst to methane to avoid the rapid deactivation associated with high concentrations of methane, by, for example, adding deactivated particulate solid to the combustor downstream of the supplemental fuel stream relative to a flow direction of the supplemental fuel stream and adding reactivated particulate solid to the combustor upstream of the supplemental fuel stream relative to the flow direction of the supplemental fuel stream may improve the catalytic activity of the total catalyst improving the dehydrogenation performance of the reactor system as a whole.

Although some aspects of the present disclosure may be identified herein as preferred or particularly advantageous, it is contemplated that the present disclosure is not limited to these aspects.

It is noted that the various details described in this disclosure should not be taken to imply that these details relate to elements that are essential components of the various embodiments described in this disclosure, even in cases where a particular element is illustrated in each of the drawings that accompany the present description. Unless specifically identified as such, no feature disclosed and described herein should be construed as "essential". Contemplated embodiments of the present technology include those that include some or all of the features of the appended claims.

For the purposes of describing and defining the present disclosure it is noted that the term "about" are utilized in this disclosure to represent the inherent degree of uncertainty that may be attributed to any quantitative comparison, value, measurement, or other representation. The term "about" are also utilized in this disclosure to represent the degree by which a quantitative representation may vary from a stated reference without resulting in a change in the basic function of the subject matter at issue.

In relevant cases, where a composition is described as "comprising" one or more elements, embodiments of that composition "consisting of" or "consisting essentially of" those one or more elements is contemplated herein.

It should be appreciated that compositional ranges of a chemical constituent in a stream or in a reactor should be appreciated as containing, in some embodiments, a mixture of isomers of that constituent. For example, a compositional range specifying butene may include a mixture of various isomers of butene. It should be appreciated that the examples supply compositional ranges for various streams, and that the total amount of isomers of a particular chemical composition can constitute a range.

It is noted that one or more of the following claims and the detailed description utilize the terms "where" or "wherein" as a transitional phrase. For the purposes of defining the present technology, it is noted that this term is introduced in the claims as an open-ended transitional phrase that is used to introduce a recitation of a series of characteristics of the structure and should be interpreted in like manner as the more commonly used open-ended preamble term "comprising."

It should be understood that any two quantitative values assigned to a property may constitute a range of that property, and all combinations of ranges formed from all stated quantitative values of a given property are contemplated in this disclosure. Where multiple ranges for a quantitative value are provided, these ranges may be combined to form a broader range, which is contemplated in the embodiments described herein.

As would be understood in the context of the term as used herein, the term "passing" may include directly passing a substance between two portions of the disclosed system and, in some other instances, to mean indirectly passing a substance between two portions of the disclosed system. For example, indirect passing may include steps where the named substance passes through an intermediate operations unit, valve, sensor, etc.

## Claims

1. A method for producing olefinic compounds, the method comprising:
contacting a feed stream comprising one or more hydrocarbons with a particulate solid catalyst in a reactor, wherein in the reactor:
the one or more hydrocarbons are dehydrogenated to form one or more products comprising one or more olefinic compounds; and
at least a portion of the particulate solid catalyst is deactivated;
passing at least a portion of the deactivated particulate solid catalyst to a combustor, wherein in the combustor:
a supplemental fuel stream enters the combustor through a supplemental fuel distributor; and
the supplemental fuel stream is combusted to heat at least a portion of the deactivated particulate solid catalyst;
passing at least a portion of the heated deactivated particulate solid catalyst to an oxygen treatment zone to produce a reactivated particulate solid catalyst;
passing at least a portion of the reactivated particulate solid catalyst back to the combustor, wherein the deactivated particulate solid catalyst enters the combustor downstream of the supplemental fuel stream relative to a flow direction of the supplemental fuel stream and the reactivated particulate solid catalyst enters the combustor upstream of the supplemental fuel stream relative to the flow direction of the supplemental fuel stream; and
passing at least a portion of the reactivated particulate solid catalyst to the reactor.

2. The method of claim 1, wherein the reactivated particulate solid catalyst and the deactivated particulate solid catalyst are passed to the combustor through a particulate solids distributor that separately passes the reactivated particulate solid catalyst and deactivated particulate solid catalyst into the combustor.

3. The method of claim 2, wherein the particulate solids distributor extends into the combustor through a bottom end of the combustor.

4. The method of claim 3, wherein the particulate solids distributor passes the deactivated particulate solid catalyst into the combustor above the supplemental fuel distributor and passes the reactivated particulate solid catalyst into the combustor below the supplemental fuel distributor.

5. The method of any preceding claim, wherein the combustor operates as a fast fluidized, turbulent or bubbling bed.

6. The method of any preceding claim, wherein the temperature of the deactivated particulate solid catalyst passed to the combustor is from 580 °C to 800 °C.

7. The method of any preceding claim, wherein the temperature of the reactivated particulate solid catalyst passed to the combustor is from 680 °C to 900 °C.

8. The method of any preceding claim, wherein in the oxygen treatment zone the heated deactivated particulate solid catalyst is exposed to an oxygen-containing gas to produce a reactivated particulate solid catalyst.

9. The method of claim 8, wherein the heated deactivated particulate solid catalyst is exposed to the oxygen-containing gas in the oxygen treatment zone for from 30 seconds to 20 minutes.

10. The method of any preceding claim, wherein the particulate solid catalyst comprises one or both of a dehydrogenation catalyst material and an oxygen-carrier material.

11. The method of claim 10, wherein the dehydrogenation catalyst material and the oxygen-carrier material are contained in the same particles of the particulate solid catalyst.

12. The method of any preceding claim, wherein the supplemental fuel stream comprises hydrogen, methane, ethane, propane, natural gas, or combinations thereof.

13. The method of any preceding claim, wherein coke forms on the deactivated particulate solid catalyst in the reactor and at least a portion of the coke on the deactivated particulate solid catalyst is reacted in the combustor.

14. The method of any preceding claim, wherein the particulate solid catalyst is a Geldart A or Geldart B particulate.

15. The method of any preceding claim, wherein the one or more hydrocarbons comprise propane and the one or more olefinic compounds comprise propylene.

## Patentansprüche

1. Verfahren zum Herstellen olefinischer Verbindungen, wobei das Verfahren umfasst:
Inkontaktbringen eines Einsatzstroms, der einen oder mehrere Kohlenwasserstoffe umfasst, mit einem teilchenförmigen festen Katalysator in einem Reaktor, wobei in dem Reaktor:
der eine oder die mehreren Kohlenwasserstoffe dehydriert werden, um ein oder mehrere Produkte zu bilden, die eine oder mehrere olefinische Verbindungen umfassen; und
mindestens ein Teil des teilchenförmigen festen Katalysators deaktiviert wird;
Leiten mindestens eines Teils des deaktivierten teilchenförmigen festen Katalysators zu einer Brennkammer, wobei in der Brennkammer:
ein zusätzlicher Brennstoffstrom durch einen zusätzlichen Brennstoffverteiler in die Brennkammer eintritt; und
der zusätzliche Brennstoffstrom verbrannt wird, um mindestens einen Teil des deaktivierten teilchenförmigen festen Katalysators zu erhitzen;
Leiten mindestens eines Teils des erhitzten deaktivierten teilchenförmigen festen Katalysators in eine Sauerstoffbehandlungszone, um einen reaktivierten teilchenförmigen festen Katalysator zu erzeugen;
Leiten mindestens eines Teils des reaktivierten teilchenförmigen festen Katalysators zurück zu der Brennkammer, wobei der deaktivierte teilchenförmige feste Katalysator stromabwärts des zusätzlichen Brennstoffstroms relativ zu einer Strömungsrichtung des zusätzlichen Brennstoffstroms in die Brennkammer eintritt und der reaktivierte teilchenförmige feste Katalysator stromaufwärts des zusätzlichen Brennstoffstroms relativ zu der Strömungsrichtung des zusätzlichen Brennstoffstroms in die Brennkammer eintritt; und
Leiten mindestens eines Teils des reaktivierten teilchenförmigen festen Katalysators zu dem Reaktor.

2. Verfahren nach Anspruch 1, wobei der reaktivierte teilchenförmige feste Katalysator und der deaktivierte teilchenförmige feste Katalysator durch einen Verteiler für teilchenförmige Feststoffe zu der Brennkammer geleitet werden, der den reaktivierten teilchenförmigen festen Katalysator und den deaktivierten teilchenförmigen festen Katalysator separat in die Brennkammer leitet.

3. Verfahren nach Anspruch 2, wobei sich der Verteiler für teilchenförmige Feststoffe durch ein unteres Ende der Brennkammer in die Brennkammer erstreckt.

4. Verfahren nach Anspruch 3, wobei der Verteiler für teilchenförmige Feststoffe den deaktivierten teilchenförmigen festen Katalysator oberhalb des zusätzlichen Brennstoffverteilers in die Brennkammer leitet und den reaktivierten teilchenförmigen festen Katalysator unterhalb des zusätzlichen Brennstoffverteilers in die Brennkammer leitet.

5. Verfahren nach einem der vorstehenden Ansprüche, wobei die Brennkammer als schnelle, turbulente oder blasenbildende Wirbelschicht arbeitet.

6. Verfahren nach einem der vorstehenden Ansprüche, wobei die Temperatur des deaktivierten teilchenförmigen festen Katalysators, der zu der Brennkammer geleitet wird, von 580 °C bis 800 °C beträgt.

7. Verfahren nach einem der vorstehenden Ansprüche, wobei die Temperatur des reaktivierten teilchenförmigen festen Katalysators, der zu der Brennkammer geleitet wird, von 680 °C bis 900 °C beträgt.

8. Verfahren nach einem der vorstehenden Ansprüche, wobei in der Sauerstoffbehandlungszone der erhitzte deaktivierte teilchenförmige feste Katalysator einem sauerstoffhaltigen Gas ausgesetzt wird, um einen reaktivierten teilchenförmigen festen Katalysator zu erzeugen.

9. Verfahren nach Anspruch 8, wobei der erhitzte deaktivierte teilchenförmige feste Katalysator dem sauerstoffhaltigen Gas in der Sauerstoffbehandlungszone 30 Sekunden bis 20 Minuten lang ausgesetzt wird.

10. Verfahren nach einem der vorstehenden Ansprüche, wobei der teilchenförmige feste Katalysator eines oder beide von einem Dehydrierungskatalysatormaterial und einem Sauerstoffträgermaterial umfasst.

11. Verfahren nach Anspruch 10, wobei das Dehydrierungskatalysatormaterial und das Sauerstoffträgermaterial in denselben Partikeln des teilchenförmigen festen Katalysators enthalten sind.

12. Verfahren nach einem der vorstehenden Ansprüche, wobei der zusätzliche Brennstoffstrom Wasserstoff, Methan, Ethan, Propan, Erdgas oder Kombinationen davon umfasst.

13. Verfahren nach einem der vorstehenden Ansprüche, wobei sich Koks auf dem deaktivierten teilchenförmigen festen Katalysator in dem Reaktor bildet und mindestens ein Teil des Kokses auf dem deaktivierten teilchenförmigen festen Katalysator in der Brennkammer umgesetzt wird.

14. Verfahren nach einem der vorstehenden Ansprüche, wobei der teilchenförmige feste Katalysator ein Geldart-A- oder Geldart-B-Partikel ist.

15. Verfahren nach einem der vorstehenden Ansprüche, wobei der eine oder die mehreren Kohlenwasserstoffe Propan umfassen und die eine oder die mehreren olefinischen Verbindungen Propylen umfassen.

## Revendications

1. Procédé permettant de produire des composés oléfiniques, le procédé comprenant :
la mise en contact d'un courant d'alimentation comprenant un ou plusieurs hydrocarbures avec un catalyseur solide particulaire dans un réacteur, dans lequel, dans le réacteur :
le ou les hydrocarbures sont déshydrogénés pour former un ou plusieurs produits comprenant un ou plusieurs composés oléfiniques ; et
au moins une partie du catalyseur solide particulaire est désactivée ;
le passage d'au moins une partie du catalyseur solide particulaire désactivé vers une chambre de combustion, dans lequel, dans la chambre de combustion :
un courant de combustible supplémentaire pénètre dans la chambre de combustion à travers un distributeur de combustible supplémentaire ; et
le courant de combustible supplémentaire est brûlé pour chauffer au moins une partie du catalyseur solide particulaire désactivé ;
le passage d'au moins une partie du catalyseur solide particulaire désactivé chauffé vers une zone de traitement à l'oxygène pour produire un catalyseur solide particulaire réactivé ;
le passage d'au moins une partie du catalyseur solide particulaire réactivé en retour dans la chambre de combustion, dans lequel le catalyseur solide particulaire désactivé pénètre dans la chambre de combustion en aval du courant de combustible supplémentaire par rapport à une direction d'écoulement du courant de combustible supplémentaire et le catalyseur solide particulaire réactivé pénètre dans la chambre de combustion en amont du courant de combustible supplémentaire par rapport à la direction d'écoulement du courant de combustible supplémentaire ; et
le passage d'au moins une partie du catalyseur solide particulaire réactivé dans le réacteur.

2. Procédé selon la revendication 1, dans lequel le catalyseur solide particulaire réactivé et le catalyseur solide particulaire désactivé sont amenés à passer dans la chambre de combustion à travers un distributeur de matière solide particulaire qui fait passer séparément le catalyseur solide particulaire réactivé et le catalyseur solide particulaire désactivé dans la chambre de combustion.

3. Procédé selon la revendication 2, dans lequel le distributeur de matière solide particulaire s'étend dans la chambre de combustion à travers une extrémité inférieure de la chambre de combustion.

4. Procédé selon la revendication 3, dans lequel le distributeur de matière solide particulaire fait passer le catalyseur solide particulaire désactivé dans la chambre de combustion au-dessus du distributeur de combustible supplémentaire et fait passer le catalyseur solide particulaire réactivé dans la chambre de combustion en dessous du distributeur de combustible supplémentaire.

5. Procédé selon l'une quelconque des revendications précédentes, dans lequel la chambre de combustion fonctionne comme un lit fluidisé rapide, turbulent ou bouillonnant.

6. Procédé selon l'une quelconque des revendications précédentes, dans lequel la température du catalyseur solide particulaire désactivé amené à passer dans le dispositif de combustion va de 580 °C à 800 °C.

7. Procédé selon l'une quelconque des revendications précédentes, dans lequel la température du catalyseur solide particulaire réactivé amené à passer dans la chambre de combustion va de 680 °C à 900 °C.

8. Procédé selon l'une quelconque des revendications précédentes, dans lequel, dans la zone de traitement à l'oxygène, le catalyseur solide particulaire désactivé chauffé est exposé à un gaz contenant de l'oxygène pour produire un catalyseur solide particulaire réactivé.

9. Procédé selon la revendication 8, dans lequel le catalyseur solide particulaire désactivé chauffé est exposé au gaz contenant de l'oxygène dans la zone de traitement à l'oxygène pendant 30 secondes à 20 minutes.

10. Procédé selon l'une quelconque des revendications précédentes, dans lequel le catalyseur solide particulaire comprend l'un ou les deux parmi un matériau catalyseur de déshydrogénation et un matériau porteur d'oxygène.

11. Procédé selon la revendication 10, dans lequel le matériau catalyseur de déshydrogénation et le matériau porteur d'oxygène sont contenus dans les mêmes particules du catalyseur solide particulaire.

12. Procédé selon l'une quelconque des revendications précédentes, dans lequel le courant de combustible supplémentaire comprend de l'hydrogène, du méthane, de l'éthane, du propane, du gaz naturel, ou des combinaisons de ceux-ci.

13. Procédé selon l'une quelconque des revendications précédentes, dans lequel du coke se forme sur le catalyseur solide particulaire désactivé dans le réacteur et au moins une partie du coke sur le catalyseur solide particulaire désactivé est mise à réagir dans la chambre de combustion.

14. Procédé selon l'une quelconque des revendications précédentes, dans lequel le catalyseur solide particulaire est une matière particulaire de Geldart A ou Geldart B.

15. Procédé selon l'une quelconque des revendications précédentes, dans lequel le ou les hydrocarbures comprennent du propane et le ou les composés oléfiniques comprennent du propylène.
